# EUROPÄISCHE PATENTSCHRIFT

(11) **EP 0 643 294 B1**
(45) Veröffentlichungstag und Bekanntmachung des Hinweises auf die Patenterteilung: **14.10.1998**
(21) Anmeldenummer: 94112608.8
(22) Anmeldetag: 12.08.1994
(51) Int. Cl.: G01N 21/89, G01N 33/36

(54) **Verfahren und Vorrichtung zur Detektion von Fremdstoffen in einem textilen Prüfgut**
Method and device for the detection of foreign matter in textile materials
Procédé et dispositif de détection des corps étrangers dans un échantillon textile

(30) Priorität: 09.09.1993 CH 2705/93
(43) Veröffentlichungstag der Anmeldung: 15.03.1995
(73) Patentinhaber: ZELLWEGER LUWA AG, 8610 Uster (CH)
(72) Erfinder: Aeppli, Kurt, CH-8610 Uster (CH)
(74) Vertreter: Ellenberger, Maurice

(56) Entgegenhaltungen:
- EP-A- 0 197 763
- EP-A- 0 553 445

## Beschreibung

Verfahren zur Detektion von Fremdstoffen in einem textilen Prüfgut von der Art eines Garnes, Vorgarnes oder Bandes, bei welchem das Prüfgut mit Licht beaufschlagt, das vom Prüfgut reflektierte Licht gemessen und aus einer Aenderung des reflektierten Lichts auf das Vorhandensein eines Fremdstoffs geschlossen wird.

Bei einem aus der EP-A-0 197 763 bekannten Verfahren dieser Art ist ein das Prüfgut in der Art eines Führungsschlitzes umgebender Hintergrund vorgesehen, welcher ebenfalls mit Licht beaufschlagt ist. Der Hintergrund ist dabei so auf das Prüfgut abgestimmt, dass die Gesamtmenge des vom Prüfgut reflektierten und des vom Hintergrund herrührenden Lichts von den Abmessungen und der Dichte des Prüfguts und von der Verteilung der Fasern innerhalb von diesem unabhängig ist. Auf diese Weise soll es möglich sein, dass eine Veränderung im reflektierten Licht einen Fremdstoff anzeigt und nicht eine Veränderung in den Dimensionen, in der Dichte oder in der Faserverteilung im Prüfgut.

Abgesehen davon, dass bei diesem Verfahren bei jedem Wechsel der Art oder des Typs des Prüfguts relativ aufwendige Einstellungsarbeiten zur Anpassung des Hintergrunds an das Prüfgut erforderlich sind, ist das Verfahren auch sehr empfindlich auf Verschmutzung und Alterung des Hintergrunds. Und beides sind Phänomene, die sich in einem Textilbetrieb nicht nur nicht vermeiden lassen, sondern dort sogar häufig auftreten.

Durch die Erfindung soll nun ein Verfahren angegeben werden, welches keine spezielle Abstimmung des Hintergrunds auf das Prüfgut erfordert, und bei dem daher die genannten aufwendigen Einstellungsarbeiten nicht erforderlich sind.

Diese Aufgabe wird erfindungsgemäss dadurch gelöst, dass das Prüfgut zur Detektion von dunkleren Fremdstoffen als das Prüfgut vor hellem und zur Detektion von helleren Fremdstoffen vor dunklem Hintergrund auf einen Sensor abgebildet und dessen Signal mit einstellbaren Grenzwerten verglichen, und dass im ersten Fall eine Unterschreitung und im zweiten Fall eine Ueberschreitung des jeweiligen Grenzwerts durch das Signal als Vorhandensein des gesuchten Fremdstoffs interpretiert wird.

Das erfindungsgemässe Verfahren basiert somit darauf, dass Fremdstoffe zum ganz überwiegenden Teil entweder heller oder dunkler als das Prüfgut sind und daher durch Abbildung vor einem kontrastierenden Hintergrund relativ einfach erfasst werden können.

Die Erfindung betrifft weiter eine Vorrichtung zur Durchführung des genannten Verfahrens, mit Mitteln zur Beleuchtung des Prüfguts und mit einem lichtempfindlichen Sensor.

Die erfindungsgemässe Vorrichtung ist dadurch gekennzeichnet, dass der Sensor durch einen Zeilensensor gebildet und dass eine Beleuchtung für einen Hintergrund vorgesehen ist, die mit einer Steuerstufe verbunden ist, die die Beleuchtung abwechselnd ein- und ausschaltet.

Der Zeilensensor hat den Vorteil, dass das Bildfeld in eine Vielzahl von Elementen aufgelöst wird, so dass allfällige Fremdstoffe, die sich nur über einen kleinen Teil des Durchmessers des Prüfguts erstrecken, in einem oder mehreren Bildelementen einen starken Kontrast ergeben und daher gut detektierbar sind. Vorrichtungen mit einem Sensor, der nur aus einem einzigen Element besteht, liefern hingegen in diesem Fall nur einen sehr geringen Kontrast und sind in ihrer Empfindlichkeit stark eingeschränkt. Gegenüber Systemen mit Bildverarbeitung beschränkt sich die Signalauswertung auf eine simple Schwellwertdetektion, so dass der Hardware- und der Softwareaufwand minimal sind.

Eine bevorzugte Ausführungsform der erfindungsgemässen Vorrichtung ist dadurch gekennzeichnet, dass der Hintergrund durch eine Mattscheibe gebildet und dass eine Lichtquelle für die wahlweise Beleuchtung der Mattscheibe vorgesehen ist.

Nachfolgend wird die Erfindung anhand eines Ausführungsbeispiels und der Zeichnungen näher erläutert; es zeigt:
- Fig. 1: eine schematische Darstellung einer erfindungsgemässen Vorrichtung,
- Fig. 2: ein Blockschaltbild der Schaltung der Vorrichtung von Fig. 1; und
- Fig. 3,4: Diagramme zur Funktionserläuterung.

Die in Fig, 1 dargestellte Vorrichtung zur Detektion von Fremdstoffen in einem langgestreckten textilen Prüfgut, insbesondere in Garnen, besteht im wesentlichen aus einer Beleuchtungseinheit 1, in der der zu untersuchende Faden F vertikal zur Bildebene geführt ist, aus einem Objektiv 2 und aus einem Zeilensensor 3, auf den das Objektiv 2 den Faden F abbildet.

Die Beleuchtungseinheit 1 enthält darstellungsgemäss eine auf der optischen Achse A der Vorrichtung liegende und den Hintergrund für die Abbildung des Fadens F bildende Mattscheibe 4, eine Auflicht-Beleuchtung 5 zur Beleuchtung des Fadens F von schräg vorne, eine Rücklicht-Beleuchtung 6 zur Beleuchtung des Fadens F von schräg hinten und eine Durchlicht-Beleuchtung 7 zur Beleuchtung der Mattscheibe 4 von hinten. Die Beleuchtung des Fadens F mit Auf- und Rücklicht lässt den Faden möglichst gleichmässig erscheinen, wobei das Rücklicht insbesondere die Randzonen des Fadens F, die sonst zu dunkel erscheinen würden, aufhellt.

Durch die Durchlicht-Beleuchtung 7 erfolgt eine möglichst gleichmässige Beleuchtung der Mattscheibe 4, so dass bei nicht vorhandenem Faden F die Signale aller Fotodioden des Zeilensensors 3 etwa gleich sind. Bei Beleuchtung der Mattscheibe 4 mit der Durchlicht-Beleuchtung 7 läuft der Faden F vor einem hellen Hintergrund, wodurch Fremdstoffe, die dunkler sind als der Faden, durch den Zeilensensor 3 erfasst werden können. Zur Detektion von Fremdstoffen, die heller sind als der Faden F, wird die Durchlicht-Beleuchtung 7 abgeschaltet. Für die Erfassung von helleren und von dunkleren Fremdstoffen wird der Hintergrund des Fadens F abwechselnd nicht beleuchtet und beleuchtet, also die Durchlicht-Beleuchtung 7 abwechselnd aus- und eingeschaltet. Dabei wird die Taktfrequenz so an die Fadengeschwindigkeit angepasst, dass die jeweils erfassten Garnstücke einander überlappen.

Die Verwendung des Zeilensensors 3 ermöglicht es, nur die vom Kern des betrachteten Fadens F herrührenden Signalanteile auszuwerten, wodurch der Einfluss von Aenderungen des Durchmessers des verursachten Fadens auf das Sensorsignal ausgeschaltet ist. Beispielsweise hat der Zeilensensor 128 Pixel, so dass das Bildfeld in 128 Elemente aufgelöst wird. Allfällige Fremdstoffe, die sich nur über einen kleinen Teil des Fadendurchmessers erstrekken, ergeben in einem oder mehreren der Bildelemente einen grossen Kontrast und werden daher sicher detektiert.

Als Beleuchtungselemente werden Leuchtdioden, beispielsweise solche einer bestimmten Farbe wie grün oder rot, verwendet, oder falls deren Licht für die verlangte Fadenschwindigkeit nicht ausreicht, Laser, Blitzlampen oder Glühlampen. Es kann auch eine Leuchtdioden-Multichipanordnung mit einer speziellen Beleuchtungsoptik verwendet und im Pulsbetrieb betrieben werden.

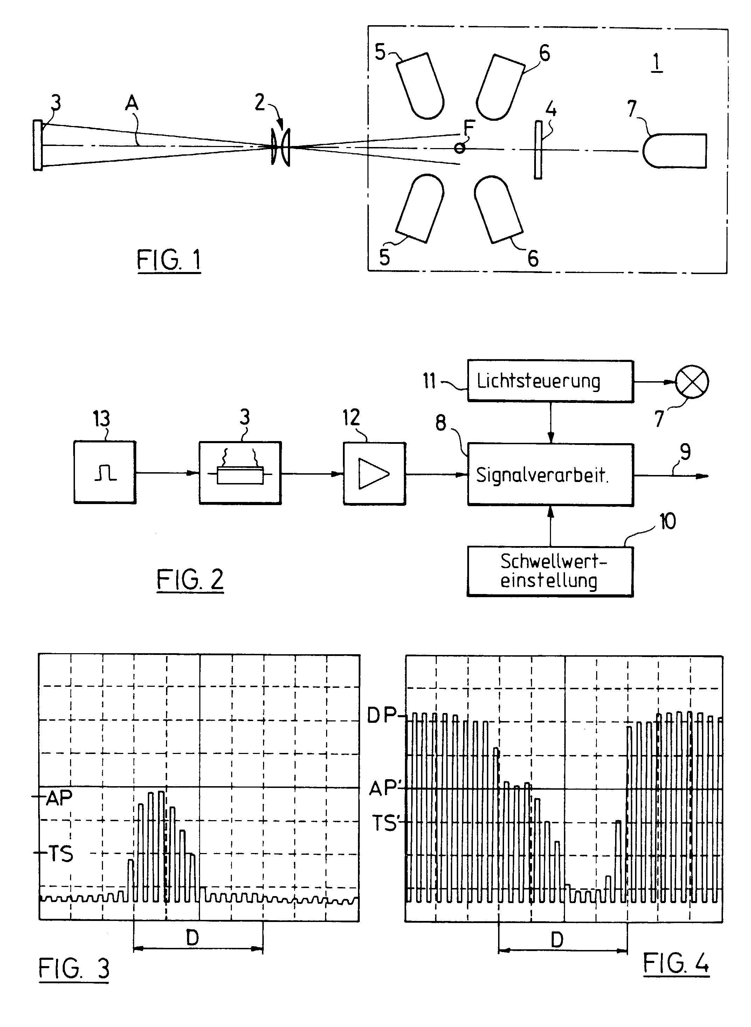

Gemäss Fig. 2 enthält die Schaltung der in Fig. 1 dargestellten Vorrichtung eine Verarbeitungsstufe 8, in der im wesentlichen zwei einstellbare Schwellwerte, einer für hellere und einer für dunklere Fremdstoffe, gespeichert sind. Die Verarbeitungsstufe 8 gibt bei Ueberschreiten des Schwellwerts für die helleren und bei Unterschreiten des Schwellwerts für die dunkleren Fremdstoffe über eine Leitung 9 jeweils ein Fremdstoffsignal ab, das dann entsprechende Massnahmen, wie beispielsweise einen Reinigerschnitt auslöst. Die beiden Schwellwerte sind über eine mit der Verarbeitungsstufe 8 verbundene Einstellstufe 10 einstellbar. Das abwechselnde Ein- und Ausschalten der Durchlicht-Beleuchtung 7 ist von einer Steuerstufe 11 geregelt, die ausserdem noch mit der Verarbeitungsstufe 8 verbunden ist und für jeden Beleuchtungszustand der Durchlicht-Beleuchtung 7 in der Verarbeitungsstufe 8 jeweils den zugehörigen Schwellwertschalter aktiviert. An einem weiteren Eingang der Verarbeitungsstufe 8 liegt der Ausgang eines das Signal des Zeilensensors 3 verstärkenden Verstärkers 12. Der zeitliche Ablauf der Operationen des Zeilensensors 3 ist von einem Taktgenerator 13 gesteuert.

Die Figuren 3 und 4 zeigen Beispiele für die Detektion von Fremdstoffen mit der in den Fig. 1 und 2 dargestellten Vorrichtung beziehungsweise Schaltung und zwar anhand des vom Zeilensensor 3 bei der Untersuchung eines aus einem weissen und aus einem schwarzen Garn bestehenden Zwirns gelieferten Signals. In den Diagrammen ist auf der Abszisse die Richtung quer zum Zwirn und auf der Ordinate die Signalamplitude aufgetragen. Der auf dem Zeilensensor 3 dem Zwirndurchmesser entsprechende Bereich ist mit den Bezugszeichen D bezeichnet.

Fig. 3 zeigt das Sensorsignal bei ausgeschalteter Durchlicht-Beleuchtung 7, also bei dunklem Hintergrund. Der schwarze Faden gibt in der zweiten Hälfte des Zwirndurchmessers praktisch kein Signal, wogegen der weisse Faden in der ersten Hälfte ein deutliches Fremdstoffsignal liefert. In der Figur bezeichnet AP den Auflichtpegel und TS die Triggerschwelle für Ueberschreitungen.

Fig. 4 zeigt das Sensorsignal bei eingeschalteter Durchlicht-Beleuchtung 7, also bei hellem Hintergrund. Hier liefert der schwarze Faden ein Fremdstoffsignal, das als tiefer Einbruch erkennbar ist. Die Signalanteile mit dem Pegel DP ausserhalb des dem Zwirndurchmesser entspechenden Bereichs D sind durch die Durchlicht-Beleuchtung 7 verursacht, die bei dem gezeigten Beispiel stärker ist als Auflicht- und Rücklicht-Beleuchtung (Pegel AP') zusammen. Somit ist das Verhältnis der Beleuchtung so gewählt, dass der weisse Faden weniger Signal abgibt als die leere Fadenführung ohne Faden. TS' bezeichnet die Triggerschwelle für Unterschreitungen.

Im "leeren" Zustand der Vorrichtung ohne Faden kann mit eingeschalteter Durchlicht-Beleuchtung 7 für jedes Element der Diodenzeile dessen Intensitätswert analog oder digital abgespeichert werden. Im Betrieb werden die Messungen mit dem Prüfgut F elementweise auf die abgespeicherten Werte bezogen; der Offset wird also elementweise subtrahiert. Diese Betriebsweise hat den Vorteil, dass Inhomogenitäten in der Hintergrundbeleuchtung kompensiert und Fehler durch einzelne Diodenzeilen-Elemente mit kleinerer Fotoempfindlichkeit vermieden werden.

Ausserdem wird auch eine sich langsam aufbauende Verschmutzung des Messfeldes, die zu zusätzlichen Inhomogenitäten führt, kompensiert. Im Durchlicht wird im leeren Zustand aus der Lichtintensität und/oder dem Helligkeitsprofil ein Wert für die Verschmutzung der lichtexponierten Teile der Vorrichtung abgeleitet und ein entsprechender Alarm gesetzt.

Die Auflicht- und/oder Rücklicht-Beleuchtung 5 bzw. 6 wird vorzugsweise vor Inbetriebnahme durch elementweise Abspeicherung der Lichtintensitätsmaxima beim Einfahren eines möglichst garnähnlichen Prüfkörpers geeicht. Die Offsetkompensation erfolgt so, wie gerade für die Durchlicht-Beleuchtung 7 beschrieben.

Die anhand der Figuren 1 und 2 beschriebene Vorrichtung zur Detektion von Verunreinigungen in einem Garn ist als kompakter Messkopf ausgebildet und wird vorzugsweise in Kombination mit einem elektronischen Garnreiniger verwendet (siehe dazu die EP-B-0 197 763), dessen Schneidvorrichtung zusätzlich zum Messkopf des Reinigers auch vom Messkopf für die Verunreinigungen angesteuert ist.

Der Messkopf kann so ausgebildet sein, dass mit einer einzigen optischen Abtastvorrichtung mehrere Funktionen ausgeführt werden können, also beispielsweise Garnreinigung, Haarigkeitsmessung und Fremdfasererkennung. Ein dafür geeigneter Messkopf mit einem Zeilensensor ist in der CH-A-643 060 beschrieben. Bei diesem Messkopf werden die einzelnen Fotoelemente des Zeilensensors abgetastet, wodurch eine zeitliche Auflösung des Durchmesser- oder Querschnittsbildes des untersuchten Fadens in Form einer vielfach für die weitere digitale Messwertverarbeitung direkt einsetzbaren Impulsfolge erreicht wird. Ebenso ist es möglich, mit einem kombinierten kapazitiv/optischen Messorgan der in der EP-A-0 401 600 beschriebenen Art kapazitiv die Garnfehler für die Garnreinigung und optisch die Haarigkeit und die für Fremdfasern repräsentative Reflexion zu messen.

Es ist auch eine Ausführung als integrierter Photo-ASIC mit Steuerelektronik zur Austastung und/oder mit einer Auswertelektronik möglich. Hier wäre sogar eine parallele Verarbeitung der Diodenzeilen-Signale möglich, bei der jedes Element einen eigenen Verstärker und eine eigene Offsetkompensation einschliesslich analoger Einspeicherung der Offsets hätte. Der Schwellwert für die Fremdfasererkennung wird in diesem Fall für alle Elemente gemeinsam gesetzt.

## Patentansprüche

1. Verfahren zur Detektion von Fremdstoffen in einem textilen Prüfgut von der Art eines Garnes, Vorgarnes oder Bandes, bei welchem das Prüfgut mit Licht beaufschlagt, das vom Prüfgut reflektierte Licht gemessen und aus einer Aenderung des reflektierten Lichts auf das Vorhandensein eines Fremdstoffs geschlossen wird, dadurch gekennzeichnet, dass das Prüfgut (F) zur Detektion von dunkleren Fremdstoffen als das Prüfgut vor hellem und zur Detektion von helleren Fremdstoffen vor dunklem Hintergrund (4) auf einen Sensor (3) abgebildet und dessen Signal mit einstellbaren Grenzwerten verglichen, und dass im ersten Fall eine Unterschreitung und im zweiten Fall eine Ueberschreitung des jeweiligen Grenzwerts durch das Signal als Vorhandensein des gesuchten Fremdstoffs interpretiert wird.

2. Verfahren nach Anspruch 1, dadurch gekennzeichnet, dass zur Detektion von dunkleren und helleren Fremdstoffen der Hintergrund (4) abwechselnd beleuchtet und nicht beleuchtet wird.

3. Verfahren nach Anspruch 2, dadurch gekennzeichnet, dass synchron mit dem Wechsel der Beleuchtung des Hintergrunds (4) auf den jeweiligen Grenzwert umgeschaltet wird.

4. Vorrichtung zur Durchführung des Verfahrens nach Anspruch 1, mit Mitteln (5,6) zur Beleuchtung des Prüfguts und mit einem lichtempfindlichen Sensor (3), dadurch gekennzeichnet, dass der Sensor durch einen Zeilensensor (3) gebildet und dass eine Beleuchtung (7) für einen Hintergund (4) vorgesehen ist, die mit einer Steuerstufe (11) verbunden ist, die die Beleuchtung (7) abwechselnd ein- und ausschaltet.

5. Vorrichtung nach Anspruch 4, dadurch gekennzeichnet, dass der Hintergrund durch eine Mattscheibe (4) gebildet und dass eine Lichtquelle (7) für die wahlweise Beleuchtung der Mattscheibe vorgesehen ist.

6. Vorrichtung nach Anspruch 5, dadurch gekennzeichnet, dass die genannte Lichtquelle (7) an der vom Zeilensensor (3) abgewandten Seite der Mattscheibe (4) angeordnet ist.

7. Vorrichtung nach einem der Ansprüche 4 bis 6, dadurch gekennzeichnet, dass die Mittel zur Beleuchtung des Prüfguts (F) eine Auflicht- und eine Rücklicht-Beleuchtung (5 bzw. 6) enthalten.

8. Vorrichtung nach Anspruch 7, dadurch gekennzeichnet, dass die Mittel zur Beleuchtung des Prüfguts (F) durch Leuchtdioden, Laserdioden, Blitzlampen oder Glühlampen gebildet sind.

9. Vorrichtung nach Anspruch 5 oder 6, dadurch gekennzeichnet, dass das Ausgangssignal des Zeilensensors (3) über einen Verstärker (12) zu einer Verarbeitungsstufe (8) geführt ist, in welcher zwei einstellbare Grenzwerte, einer für hellere und einer für dunklere Fremdstoffe, gespeichert sind, und dass in der Verarbeitungsstufe ein Vergleich des genannten Ausgangssignals mit dem jeweiligen Grenzwert erfolgt, wobei die Umschaltung zwischen den Grenzwerten synchron mit der Umschaltung der für die Beleuchtung der Mattscheibe (4) vorgesehenen Lichtquelle (7) erfolgt.

## Claims

1. Process for detecting foreign substances in a textile test material of the yarn, roving or sliver type, in which the test material is subjected to light, the light reflected from the test material is measured and the presence of a foreign substance is concluded from a change in the reflected light, characterized in that an image of the test material (F) is projected onto a sensor (3) in front of a light background (4) for the detection of darker foreign substances than the test material and in front of a dark background (4) for the detection of lighter foreign substances, and the signal of the said sensor is compared with adjustable limit values, and in that in the first case an undershooting of the respective limit value and in the second case an overshooting of the respective limit value by the signal is interpreted as presence of the foreign substance sought.

2. Process according to Claim 1, characterized in that, for the detection of darker and lighter foreign substances, the background (4) is alternately illuminated and not illuminated.

3. Process according to Claim 2, characterized in that switching to the respective limit value is carried out synchronously with the changing of the illumination of the background (4).

4. Device for carrying out the process according to Claim 1, with means (5, 6) for illuminating the test material and with a light-sensitive sensor (3) , characterized in that the sensor is formed by a line sensor (3) and in that an illuminating means (7) is provided for a background (4), which illuminating means is connected to a conrol stage (11) which alternately switches the illuminating means (7) on and off.

5. Device according to Claim 4, characterized in that the background is formed by a ground glass screen (4) and in that a light source (7) is provided for the optional illumination of the ground glass screen.

6. Device according to Claim 5, characterized in that the said light source (7) is arranged on the side of the ground glass screen (4) facing away from the line sensor (3).

7. Device according to one of Claims 4 to 6, characterized in that the means for illuminating the test material (F) include a direct-light illuminating means (5) and a back-light illuminating means (6).

8. Device according to Claim 7, characterized in that the means for illuminating the test material (F) are formed by light-emitting diodes, laser diodes, flashlamps or incandescent lamps.

9. Device according to Claim 5 or 6, characterized in that the output signal of the line sensor (3) is passed via an amplifier (12) to a processing state (8), in which two adjustable limit values, one for lighter foreign substances and one for darker foreign substances, are stored, and in that in the processing stage a comparison of the said output signal with the respective limit value is performed, the switching between the limit values being carried out synchronously with the switching of the light source (7) provided for the illumination of the ground glass screen (4).

## Revendications

1. Procédé de détection de corps étrangers dans un échantillon textile du type de filé, mèche ou bande où l'échantillon est sollicité par de la lumière, la lumière réfléchie par l'échantillon est mesurée et à partir d'une modification de la lumière réfléchie, on conclut à la présence d'un corps étranger, caractérisé en ce que l'échantillon (F), pour la détection de corps étrangers plus sombres que l'échantillon est représenté devant un arrière-plan clair et pour la détection de corps étrangers plus clairs devant un arrière-plan sombre (4) sur un capteur (3) et le signal de celui-ci est comparé à des valeurs limites réglables et en ce que dans le premier cas une non-atteinte et dans le deuxième cas un dépassement de la valeur limite respective est interprété par le signal comme présence du corps étranger recherché.

2. Procédé selon la revendication 1, caractérisé en ce que pour la détection de corps étrangers plus sombres et plus clairs, l'arrière-plan (4) est éclairé et n'est pas éclairé alternativement.

3. Procédé selon la revendication 2, caractérisé en ce que d'une manière synchronisée avec le changement de l'éclairage de l'arrière-plan (4), il y a commutation à la valeur limite respective.

4. Dispositif pour la mise en oeuvre du procédé selon la revendication 1, avec des moyens (5, 6) pour éclairer l'échantillon et avec un capteur (3) réagissant à la lumière, caractérisé en ce que le capteur est formé par un capteur de lignes (3) et en ce qu'il est prévu un éclairage (7) pour un arrière-plan (4) qui est relié à un étage de commande (11) qui met l'éclairage (7) alternativement en et hors service.

5. Dispositif selon la revendication 4, caractérisé en ce que l'arrière-plan est formé par un verre dépoli (4) et en ce qu'il est prévu une source de lumière (7) pour l'éclairage sélectif du verre dépoli.

6. Dispositif selon la revendication 5, caractérisé en ce que la source de lumière précitée (7) est disposée au côté du verre dépoli (4) éloigné du capteur de ligne (3).

7. Dispositif selon l'une des revendications 4 à 6, caractérisé en ce que les moyens d'éclairage de l'échantillon (5) comportent un éclairage incident et un éclairage arrière (5 respectivement 6).

8. Dispositif selon la revendication 7, caractérisé en ce que les moyens pour éclairer l'échantillon (F) sont constitués par des diodes luminescentes, des diodes à laser, des lampes éclair ou des lampes à incandescence.

9. Dispositif selon la revendication 5 ou 6, caractérisé en ce que le signal de départ du capteur de lignes (3) est guidé par un amplificateur (12) vers un étage de traitement (8) dans lequel sont stockées deux valeurs limites réglables, une pour des corps étrangers plus clairs et une pour des corps étrangers plus sombres et en ce qu'a lieu dans l'étage de traitement une comparaison du signal de départ précité avec la valeur limite respective, la commutation entre les valeurs limites ayant lieu d'une manière synchronisée avec la commutation de la source de lumière (7) prévue pour l'éclairage du verre dépoli (4).
